# EUROPEAN PATENT SPECIFICATION

(11) **EP 3 484 392 B1**
(45) Date of publication and mention of the grant of the patent: **16.09.2020**
(21) Application number: 17742698.8
(22) Date of filing: 12.07.2017
(51) Int. Cl.: A61B 17/86, A61B 17/88

(54) **CANNULATED BONE SCREW**
KANÜLIERTE KNOCHENSCHRAUBE
VIS À OS CANULÉE

(30) Priority: 14.07.2016 US 201615210427; 14.07.2016 BE 201600127
(43) Date of publication of application: 22.05.2019
(73) Proprietor: Clariance, 62217 Beaurains (FR)
(72) Inventor: MAZEL, Christian, 92350 Le Plessis Robinson (FR)
(74) Representative: Godard, Xavier
(86) International application number: PCT/EP2017/067591
(87) International publication number: WO 2018/011290

(56) References cited:
- US-A1- 2011 060 373
- US-A1- 2014 194 886

## Description

### FIELD OF INVENTION

The present invention pertains to the field of bone screws. In particular, the invention relates to cannulated bone screws for the treatment of bone.

### BACKGROUND OF INVENTION

Delivery of drugs or therapeutics to bones is an often desirable but difficult to achieve process, especially if one desires to focus the delivery to the interior of a bone or to a particular area in a bone.

US 2014/0236242 teaches to use a screw for bone fixation comprising an elongate shank defining an internal longitudinal passage and at least one shank aperture in communication with the internal longitudinal passage. However, with such device, the discharge of substances within the bone is mainly distal due to the distal opening required to insert a guiding wire. Such guiding wire is necessary for minimally invasive surgery. Minimally invasive surgery, which is applied to an increasing number of cases, requires indeed guiding wires to position an implant to the implantation site.

EP 2570093 suggests inserting a plug member into the internal longitudinal passage to close the passage at the distal end after removal of the guiding wire. Such system enables radial ejection of substance by preventing distal discharge of fluid. However, insertion of the plug member requires complex handling. US2011/0060373 describes a bone screw having a threaded screw body including an interior channel extending longitudinally through the screw head and a plurality of radially-disposed delivery channels connecting the interior channel to the exterior of the screw body, each delivery channel having exterior openings.

US2014/0194886 discloses a bone screw having a shank which has a longitudinal cannula therein. A plurality of fenestrations extends outwardly from the cannula to the exterior of the screw. A bone cement may be delivered to the cannula and forced out of the fenestrations and into bone structure proximate the fenestrations.

It is thus a first object of the invention to provide a bone screw enabling discharge of substances in an oriented manner while allowing minimally invasive surgery.

The object is solved by the device as defined in the claims. The present invention especially provides a bone screw having a longitudinal axis, a through bore extending coaxially and at least three blind channels angularly-disposed around the through bore. Each blind channel is connected to the exterior of the bone screw by fenestrations. With such device, the discharge of substances may be oriented by selecting the desired blind channel.

Orienting the delivery of substance within the bone may be of importance, especially for treating the intervertebral disc. Intervertebral disc has indeed no specific vascularization. The discs of the human spine are the largest non-vascularized structures in the body. Avascular tissue nutrients are delivered by diffusion from the capillaries at the discvertebral body interface. Nutrients originate at vertebral capillaries, pass through endplate bone and cartilage, and then diffuse through annulus fibrosus. Consequently, intravenous treatments have difficulties to access the intervertebral disc. The state of the art procedure requires disc puncture and perforation of the disc annulus. However, such procedure often leads to degenerative disc disease.

It is consequently a second object of the invention to enable treatment of the intervertebral disc without puncture of the disc annulus.

The cannulated bone screw according to the invention has the advantage that it can deliver a substance inside the vertebral body precisely towards the superior or inferior endplate; such substance being subsequently diffused inside respectively the superior or inferior intervertebral disc.

In order to further promote diffusion of substances into the intervertebral disc, the present invention also provides a kit of parts as defined in the claims comprising a cannulated bone screw and at least one endplate puncturing means. The endplate puncturing means is configured to puncture the endplate of the vertebra, without perforating or damaging the annulus fibrosus. In combination with the cannulated bone screw of the invention, the endplate puncturing means ensures optimal diffusion of substance towards the disc.

Alternatively, the cannulated bone screw according to the invention may also be used with radioactive needles positioned in the blind channels, thereby providing local radiotherapy within the bone.

After the implant is inserted into the vertebral body, a catheter, inserted in a blind channel, may be connected to a pump implanted just below the surface of the skin. The patient may thereby keep the system for several hours to several days. Once the treatment is achieved, in order to access the bone screw percutaneously, it is necessary that extracting means locate the head screw easily.

It is thus a third object of the invention to provide a bone screw enabling removal by minimally invasive surgery.

The object is solved by the device as defined in the claims. In particular, the present invention provides flexible directing means secured to the screw head such that extracting means may be guided to the screw head. As the directing means are secured to the screw head, the extracting means may slide around said directing means until it abuts against the screw head for retrieval.

### SUMMARY

The present invention relates to a bone screw comprising:
- a screw body comprising a proximal end, a distal end, a longitudinal axis and a thread along all or a portion of said screw body;
- a screw head attached to the proximal end of the screw body;
- a through bore extending coaxially through said screw head and through said screw body from the proximal end up to the distal end;
- at least three blind channels angularly-disposed around the through bore; said channels extending longitudinally through said screw head and through a portion of said screw body; and each of said blind channels being configured to receive a catheter or a needle;
- a plurality of radially-disposed fenestrations; wherein each of said blind channels is connected to an exterior of said screw body through at least one fenestration.

In one embodiment, the at least three blind channels are regularly angularly-disposed. In one embodiment, the bone screw comprises four blind channels disposed at every 90°. In one embodiment, at least one of the blind channels has a different length than the other channels.

In one embodiment, the fenestrations are elongated holes. In one embodiment, the plurality of radially-disposed fenestrations is longitudinally spaced. In one embodiment, a fenestration is located at the distal end of each blind channel. In one embodiment, the at least one fenestration connecting each blind channel to an exterior of the screw body are not positioned in the same transverse plane. In one embodiment, the fenestrations are positioned between the crests of adjacent thread (i.e. between adjacent crests of the thread).

In one embodiment, the bone screw further comprises a radiopaque marker (or radiopacifier) enabling positioning, especially angular positioning, of the bone screw within the bone.

In one embodiment, the bone screw further comprises flexible directing means secured to the screw head such that extracting means may be guided to the screw head along said flexible directing means. In one embodiment, the flexible directing means are a wire, preferably a braided wire, more preferably a metallic braided wire. In one embodiment, the flexible directing means is hollow and configured to receive at least one catheter or at least one needle According to said embodiment, the flexible directing means may be a braided sleeve, preferably a metallic braided sleeve; or a coiled sleeve, preferably an elastic or metallic coiled sleeve.

In one embodiment, the lead of the thread varies along the screw body. In one embodiment, the lead of the thread is larger in the distal portion of the screw body than in the proximal portion of the screw body.

The present invention also relates to a system comprising a bone screw according to the present invention and at least one catheter, such as for example a perforated catheter, or at least one needle, such as for example a radioactive needle.

In one embodiment, the system further comprises a stopper comprising an opening for each catheter or each needle and for guiding means; said stopper being configured to be inserted in the screw head in a tight manner.

In one embodiment, the system further comprises a subcutaneous fluid pump fluidly connected to the catheter.

The present invention also relates to a kit of parts comprising a bone screw according to the present invention and at least one endplate puncturing means.

In one embodiment, the endplate puncturing means comprises at least one aperture extending therethrough; and engaging means configured to mechanically engaged the endplate of a vertebra. In one embodiment, the aperture extends from a distal end to a proximal end along a longitudinal axis or from a side opening adjacent to a distal end to a proximal end.

In one embodiment, the distal end is blunted so as to prevent disc perforation. In one embodiment, the length of the endplate puncturing means from a distal end to a proximal end along a longitudinal axis is lower than 5 mm. In one embodiment, the endplate puncturing means comprises a radiopaque marker at its distal end.

Not being part of the invention, a method of delivering a substance to an intervertebral disc; said method comprises the following steps:
implanting via transpedicular approach at least one system comprising:
   - a bone screw comprising:
      - a screw body comprising a proximal end, a distal end, a longitudinal axis and a thread along all or a portion of said screw body;
      - a screw head attached to the proximal end of the screw body;
      - a through bore extending coaxially through said screw head and through said screw body from the proximal end up to the distal end;
      - at least three blind channels angularly-disposed around the through bore; said channels extending longitudinally through said screw head and through a portion of said screw body; and each of said blind channels being configured to receive a catheter or a needle;
      - a plurality of radially-disposed fenestrations; wherein each of said blind channels is connected to an exterior of said screw body through at least one fenestration;
      and
   - at least three perforated catheters, wherein each bind channel comprises a perforated catheter.
selecting the catheter which is in the blind channel directed towards the superior or the inferior endplate; and
delivering a substance within the selected catheter such that the substance diffuses towards respectively the superior or the inferior intervertebral disc.

As an example, the method further comprises the step of implanting a fluid pump fluidly connected to the selected catheter subcutaneously.

In one example, the bone screw further comprises flexible directing means secured to the screw head such that extracting means may be guided to the screw head and the method further comprises the step of guiding extracting means along the flexible directing means to the screw head and extracting the bone screw and the at least three catheters.

In one example, the method of delivering a substance to an intervertebral disc further comprises, prior to the delivery of at least one substance within the selected catheter, the steps of:
- accessing via the selected blind channel a steerable inserter carrying an endplate puncturing means at its distal end;
- steering the steerable inserter in such a way that the inserter exits out of the bone screw through one fenestration;
- puncturing respectively the superior or the inferior endplate; and
- removing of the steerable inserter.

Not being part of the invention, the present disclosure further relates to a method of treating vertebral tumor; said method comprises the following steps:
implanting via transpedicular approach at least one system comprising:
   - a bone screw comprising:
      - a screw body comprising a proximal end, a distal end, a longitudinal axis and a thread along all or a portion of said screw body;
      - a screw head attached to the proximal end of the screw body;
      - a through bore extending coaxially through said screw head and through said screw body from the proximal end up to the distal end;
      - at least three blind channels angularly disposed around the through bore; said channels extending longitudinally through said screw head and through a portion of said screw body; and each of said blind channels being configured to receive a perforated catheter or a needle; and
      - a plurality of radially-disposed fenestrations; wherein each of said blind channels is connected to an exterior of said screw body through at least one fenestration;
      and
   - at least one radioactive needle positioned in at least one blind channel; and irradiating the vertebra.

In one example, the bone screw further comprises flexible directing means secured to the screw head such that extracting means may be guided to the screw head and the method further comprises the step of guiding extracting means along the flexible directing means to the screw head and extracting the bone screw and the at least one needle.

### DEFINITIONS

In the present invention, the following terms have the following meanings:
- The term **"about"** is used herein to mean approximately, roughly, around, or in the region of. When the term "about" is used in conjunction with a numerical range, it modifies that range by extending the boundaries above and below the numerical values set forth. In general, the term "about" is used herein to modify a numerical value above and below the stated value by a variance of 20 percent, preferably of 5 percent.
- **"Distal"** refers to the direction toward which the bone screw is advanced as the bone screw is engaged to bone and **"proximal"** refers to the direction opposite the distal direction.
- **"Flexible"** refers to the ability of an object to deform in a reversible manner.
- **"Longitudinally"** means extending in the direction of the length of a material.
- **"Substance"** refers to one or more chemical compounds that are useful when delivered to the vicinity of a bone, preferably to the vicinity of a vertebra or to the vicinity of an intervertebral disc. Substances may be chosen to help treat diseased bone or joint. Representative example substances include drugs or therapeutics such as anti-inflammatory drugs, anesthetics, antibiotics, chemotherapeutics, interleukin, bone morphogenetic proteins, bone growth factors or disc nutrients. Within the present invention, substance may be a liquid or a gel.
- **"Therapeutically effective amount"** means level or amount of agent that is aimed at, without causing significant negative or adverse side effects to the target, (1) delaying or preventing the onset of a disease; (2) slowing down or stopping the progression, aggravation, or deterioration of one or more symptoms of the disease; (3) bringing about ameliorations of the symptoms of the disease; (4) reducing the severity or incidence of the disease; or (5) curing the disease. A therapeutically effective amount may be administered prior to the onset of the disease, for a prophylactic or preventive action. Alternatively or additionally, the therapeutically effective amount may be administered after initiation of the disease, for a therapeutic action.
- Within the present invention, a **"through bore"** is a bore that goes all the way through the material. This is to distinguish it from a **"blind channel",** which does not go all the way through the material of the object.
- **"Transverse plane"** refers to a plane perpendicular to the longitudinal axis of the screw body.

### DETAILED DESCRIPTION

The following detailed description will be better understood when read in conjunction with the drawings. For the purpose of illustrating, the device is shown in the preferred embodiments. It should be understood, however that the application is not limited to the precise arrangements, structures, features, embodiments, and aspect shown. The drawings are not drawn to scale and are not intended to limit the scope of the claims to the embodiments depicted. Accordingly it should be understood that where features mentioned in the appended claims are followed by reference signs, such signs are included solely for the purpose of enhancing the intelligibility of the claims and are in no way limiting on the scope of the claims.

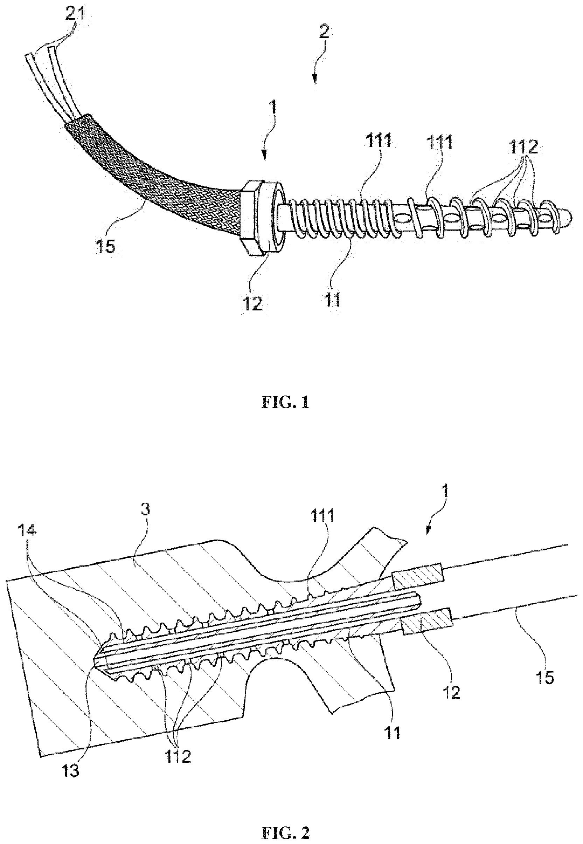

Figs. 1 to 12 show a bone screw 1 or a system 2 comprising the bone screw 1 according to the invention. As depicted in Fig. 2, the bone screw 1 comprises a screw body 11 for anchoring in the bone, e.g. a vertebra 3. Said screw body 11 comprises a proximal end, a distal end which may be shaped as a tip, a longitudinal axis and a thread 111 along all or a portion of said screw body 11. Said thread 111 is located along all or a portion of the exterior of the screw body 11. The bone screw 1 further comprises a screw head 12 attached to the proximal end of the screw body 11. The screw head 12 may be any screw head comprising engagement structure, e.g. a hexagonal screw head as depicted in Fig. 1, for engagement with a tool, e.g. inserting means and extracting means. In one embodiment, the screw head 12 is integral with the screw body 11. In one embodiment, the screw body 11 has a length ranging from 35 to 55 mm. In one embodiment, as depicted in Fig. 2, the screw body 11 is substantially cylindrical and the diameter of the screw body 11 is ranging from 5.5 mm to 6.5 mm, preferably about 6 mm.

The material from which the bone screw 1, especially the screw body 11 and the screw head 12 is made, can be any material which is usual for bone screws. In one embodiment, said material is selected from titanium, titanium alloy such as nitinol, stainless steel or any biocompatible metal alloy. In another embodiment, said material is a biocompatible plastic, such as PEEK.

According to one embodiment, the lead of the thread 111 varies along the screw body 11. According to one embodiment, the lead of the thread 111 is larger in the distal portion of the screw body 11 than in the proximal portion of the screw body 11. Said embodiment may improve anchorage of the bone screw 1 within the pedicle of a vertebra 3.

The bone screw 1 is cannulated. As shown in Figs 2, 3A and 3B, it comprises a through bore 13 extending coaxially, relative to the longitudinal axis of the screw body 11, through said screw head 12 and through said screw body 11 from the proximal end up to the distal end. The diameter of the through bore 13, and especially of the portion of the through bore extending within the screw body 11, is designed such that guiding means 22 (see Fig. 7B), commonly used for minimally invasive surgery, can be guided through the bone screw 1. In one embodiment, the diameter of the through bore 13 is ranging from 0.8 mm to 2.8 mm, preferably about 1.8 mm.

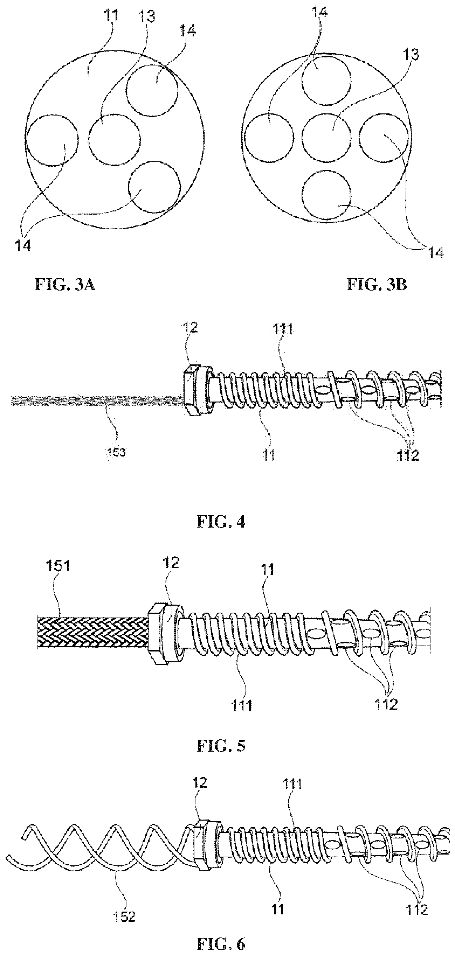

As shown in Figs. 2, 3A, 3B and 9, the bone screw 1 further comprises at least three blind channels 14 angularly (i.e. peripherally-spaced) disposed (or positioned) around the through bore 13. Said blind channels 14 extend longitudinally (i.e. parallel to the longitudinal axis of the screw body 11) through said screw head 12 and through a portion of said screw body 11. According to one embodiment, said blind channels 14 are regularly disposed around the through bore 13. According to one embodiment, as depicted in Fig. 3A, the bone screw 1 comprises three blind channels 14, preferably three blind channels 14 equally spaced around the through bore 13, i.e. at every 120°. According to one embodiment, as depicted in Fig. 3B, the bone screw 1 comprises four blind channels 14, preferably four blind channels 14 equally spaced around the through bore 13, i.e. at every 90°. As it is apparent to one skilled in the art, the bone screw 1 may comprise as many blind channels 14 as required to the skilled artisan, e.g. 3, 4, 5, 6, 7 or 8 blind channels 14. The blind channels 14 are not fluidly connected to the through bore 13 within the screw body 11.

In one embodiment, each blind channel 14 has the same diameter. The diameter of the blind channels 14, and especially of the portion of the blind channel 14 extending within the screw body 11, is designed such that a catheter or a needle can be guided therethrough. In one embodiment, the diameter of the blind channels 14 is ranging from 0.5 mm and 2.5 mm, preferably about 1.5 mm. In one embodiment, each of said blind channels 14 is configured to receive at least partially a catheter or a needle.

According to one embodiment, at least one of the blind channels 14 has a different length than the other channels. Said embodiment ensures that substances are delivered at a particular location along the screw body 11 depending of the blind channel 14. According to one embodiment, each of the blind channels 14 has a different length.

As depicted in Figs. 1, 2, 4, 5 and 6, the bone screw 1 further comprises a plurality of radially-disposed fenestrations 112. A fenestration 112 connects a blind channel 14 to an exterior of said screw body 11. In one embodiment, the bone screw 1 comprises as many fenestrations 112 as blind channels 14. In another embodiment, the bone screw 1 comprises more than one fenestration 112 for each blind channel 14. In one embodiment, the fenestrations 112 are radially-disposed from the blind channels 14 to the exterior of the screw body 11. The term radially-disposed means radially relative to the longitudinal axis of the screw body 11 in a plane transverse to said longitudinal axis.

According to one embodiment, the fenestrations 112 are circular. According to a preferred embodiment, the fenestrations 112 are elongated holes. Such embodiment maximizes the surface of the hole and prevents clogging due to healing once the bone screw 1 is inserted into the bone.

According to one embodiment, the elongated holes have an elliptic shape. In one embodiment, the minor axis of the elliptical hole is orthogonal to the longitudinal axis of the screw body 11 and has a length inferior to a quarter of the perimeter of the screw body 11 measured in a transverse plane. Such embodiment prevents that a fenestration 112 overlaps two blind channels 14. In one embodiment, the minor axis has a length equal to a fifth, a sixth, a seventh or an eighth of the perimeter of the screw body 11. In one embodiment, the major axis of the elliptical hole is parallel to the longitudinal axis of the screw body 11 and has a length equals to at least twice or at least thrice the length of the minor axis.

According to one embodiment, a fenestration 112 is located at the distal end of each blind channel 14.

According to one embodiment wherein the bone screw 1 is intended to be inserted into a vertebra 3, the fenestrations 112 are located on the distal portion of the screw body 11 such that substances may be discharged within the vertebral body and not within the pedicle. According to one embodiment, the fenestrations 112 are not located on the proximal portion of the screw body 11. In one embodiment, the length of the proximal portion of the screw body 11 is about 15 mm.

According to one embodiment, the fenestrations 112 connecting each blind channel 14 to an exterior of the screw body 11 are not positioned in the same transverse plane. According to one embodiment, the fenestrations 112 of the bone screw 1 are longitudinally spaced. According to one embodiment, the fenestrations 112 connecting adjacent blind channels 14 to an exterior of the screw body 11 are not positioned in the same transverse plane. Said embodiments ensure that substances are delivered at a particular location along the length of the screw body 11 depending of the blind channel 14. Said embodiments also strengthen the mechanical design of the bone screw 1.

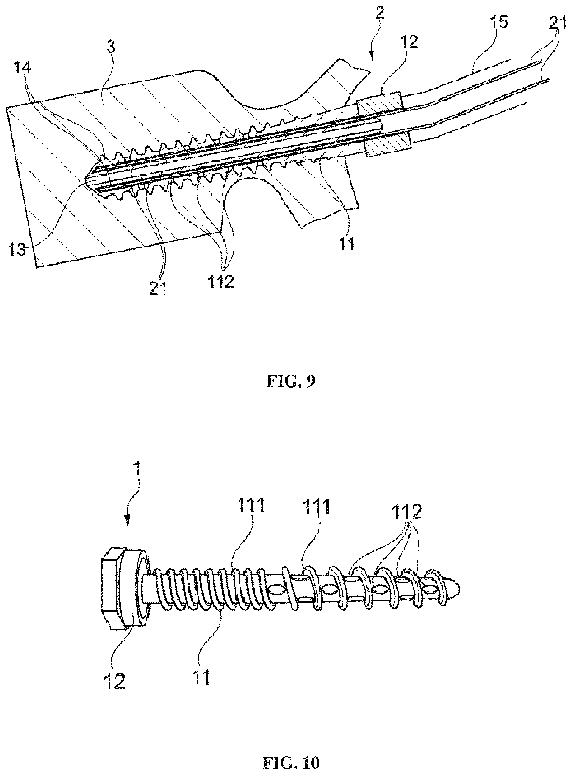

According to one embodiment, as shown in Fig. 10, the fenestrations 112 are positioned between adjacent crests of the thread.

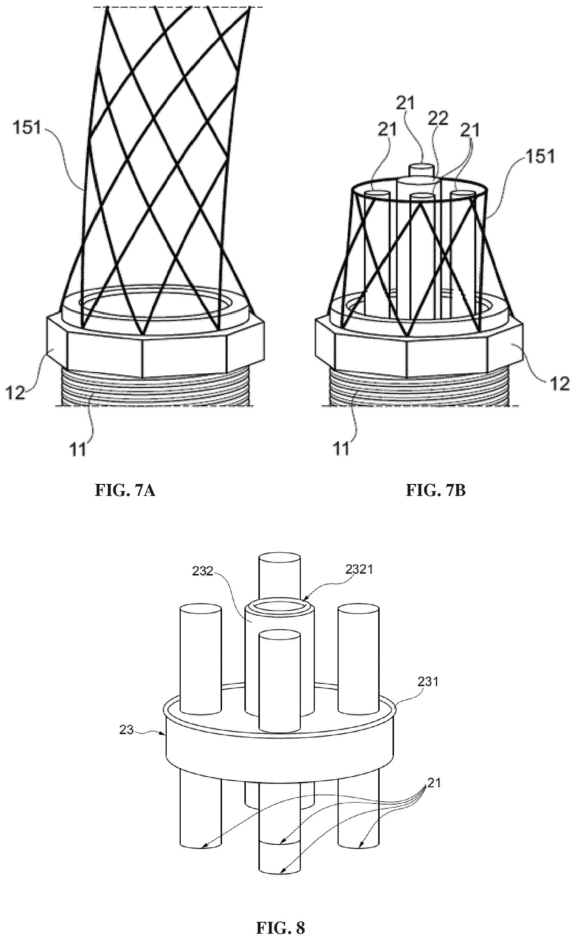

According to one embodiment, as depicted in Fig. 1, the bone screw 1 further comprises flexible directing means 15 secured to the screw head 12 such that extracting means may be guided to the screw head 12. In one embodiment, as shown in Fig. 7A, the flexible directing means 15 are secured about a proximal end of the screw head 12. The flexible directing means 15 may be secured to the screw head 12 by any means known to one skilled in the art such as for instance gluing, welding or screwing. According to one embodiment, the flexible directing means 15 have a length ranging from 10 to 15 cm, preferably about 12 cm.

According to one embodiment, as depicted in Fig. 4, the flexible directing means 15 are a flexible wire 153, preferably a flexible braided wire, more preferably a flexible metallic braided wire.

According to alternative embodiments, the directing means 15 are hollow and configured to receive at least partially at least one catheter or at least one needle, as depicted in Figs. 5 and 6. According to one embodiment, as depicted in Fig. 7B, the hollow flexible directing means 15 is configured to receive guiding means 22 and as many catheters or needles as the number of blind channels 14. According to one embodiment, the hollow flexible directing means 15 is a sleeve, preferably a cylindrical sleeve. According to one embodiment, as shown in Fig. 5, the hollow flexible directing means 15 comprises a braided sleeve 151, preferably a metallic braided sleeve. According to another embodiment, as shown in Fig. 6, the hollow flexible directing means comprises a coiled sleeve 152, preferably an elastic or metallic coiled sleeve.

According to one embodiment, the bone screw 1 further comprises a radiopaque marker (not represented) enabling positioning, especially angular positioning, of the bone screw 1 within the bone. Said embodiment enables the surgeon to choose the blind channel 14 to deliver a substance in the chosen direction.

The present invention also relates to a system 2 comprising a bone screw 1 according to the present invention.

As depicted in Fig. 1, the system 2 comprises a bone screw 1 comprising:
- a screw body 11 comprising a proximal end, a distal end, a longitudinal axis and a thread 111 along all or a portion of said screw body 11;
- a screw head 12 attached to the proximal end of the screw body 11;
- a through bore 13 extending coaxially through said screw head 12 and through said screw body 11 from the proximal end up to the distal end;
- at least three blind channels 14 angularly-disposed around the through bore 13; said channels extending longitudinally through said screw head 12 and through a portion of said screw body 11; and each of said blind channels 14 being configured to receive a catheter 21 or a needle;
- a plurality of radially-disposed fenestrations 112; wherein each of said blind channels 14 is connected to an exterior of said screw body 11 through at least one fenestration 112; and
- optionally, flexible directing means 15 secured to the screw head 12 such that extracting means may be guided to the screw head 12 along said means; and
- at least one catheter 21, preferably a perforated catheter.

The system 2 comprises a bone screw 1 and at least one catheter 21, such as a perforated catheter 21, or at least one needle, such as a radioactive needle. In one embodiment, the catheter 21 is perforated on its distal portion such that when the catheter 21 abuts the distal end of a blind channel 14, substances are discharged only in the screw body 11. In one embodiment, the system 2 comprises only one catheter 21 or needle. In one embodiment, the system 2 comprises only two catheters 21 or needles. In another embodiment, the system 2 comprises a needle or a catheter 21 in each blind channel 14.

According to one embodiment, the system 2 further comprises guiding means 22.

According to one embodiment, the system 2 further comprises a stopper 23. Said stopper 23 is configured to be inserted in the screw head 12 in a tight manner. In one embodiment, the stopper 23 comprises on its perimeter an O-ring 231ensuring tightness between the stopper 23 and the screw head 12.

According to one embodiment, the stopper 23 comprises an opening for each catheter 21 or each needle. Said openings are sized to ensure tightness between the opening and the catheter 21 or needle. Said openings comprise optionally an O-ring.

According to one embodiment, the stopper 23 comprises an opening for the guiding means 22. Said opening is sized to ensure tightness between the guiding means 22 and the stopper 23. In one embodiment, said opening comprise an O-ring. In one alternative embodiment, as depicted in Fig. 8, the stopper 23 comprises a guiding tube 232 connected to the stopper 23 in a tight manner. Said guiding tube 232 is configured to receive the guiding means 22 in a tight manner. In one embodiment, said guiding tube 232 comprises an O-ring 2321 to ensure tightness between the guiding means 22 and the guiding tube 232.

The stopper 23 according to the present invention prevents backflow from the blind channels 14 and exchanges of substances between the blind channels 14 through the screw head 12.

According to tone embodiment, the system 2 further comprises a subcutaneous fluid pump fluidly connected to the at least one catheter 21. In one embodiment, the fluid pump may be fluidly connected to each catheter 21.

According to one embodiment, the system 2 further comprises at least one substance.

The present invention also relates to a kit of parts comprising a bone screw 1 according to the present invention and an endplate puncturing means 5.

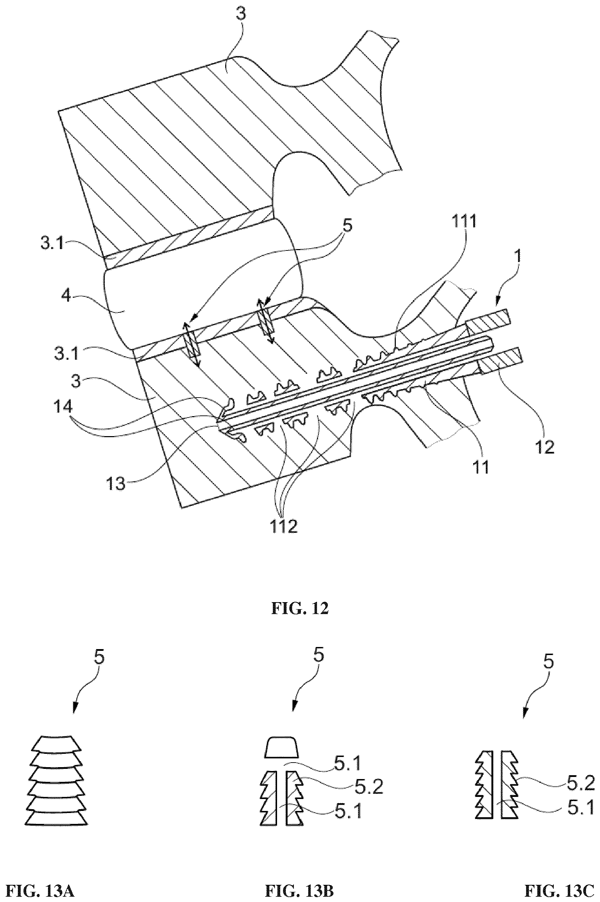

As depicted in Fig. 13, the endplate puncturing means 5 comprises at least one aperture 5.1 extending therethrough and engaging means 5.2 configured to mechanically engage the endplate of a vertebra 3. In one embodiment, the endplate puncturing means 5 is generally circular in shape.

In one embodiment as illustrated in Fig. 13C, the aperture 5.1 extends between a proximal end and a distal end along a longitudinal axis. In another embodiment depicted in Fig. 13B, the aperture 5.1 extends between a side opening adjacent to the distal end and a proximal end. In such manner, the distal end of the endplate puncturing means is closed and may not be clogged by tissue. In one embodiment, the distal end of the endplate puncturing means 5 is blunted in order to avoid perforation of the disc annulus.

In one embodiment as depicted in Fig. 13A, the engaging means 5.2 are protrusions, such as for example sawtooth protrusions, or a thread.

The aperture along the endplate puncturing means enables to establish exchange or movement of nutrients or substances between the disc and the vertebral body which is irrigated with capillaries or supply in substances with the bone screw of the invention. Consequently, the endplate puncturing anchor acts as a bypass and creates an artificial way.

According to one embodiment, the length of the endplate puncturing means 5 measured along its longitudinal axis is lower than about 5 mm, 4.5 mm, 4 mm, 3.5 mm, 3 mm, 2.5 mm 2 mm, 1.5 mm or 1 mm. The endplate puncturing means 5 may have a diameter of about 2 mm, 1.5 mm, 1 mm or 0.5 mm. The size of the aperture may be of about 0.2 mm, 0.3 mm, 0.4 mm, 0.5 mm or 0.6 mm.

According to one embodiment, the endplate puncturing means 5 comprises a radiopaque marker at the distal end of the endplate puncturing means 5. Said marker enables to ensure optimal positioning of the endplate puncturing means 5; especially flush at the disc/endplate interface.

The material of the endplate puncturing means 5 is preferably selected from metals, such as titanium and its alloys, plastics or ceramics. In one embodiment, the endplate puncturing means 5 may be bioabsorbable.

Not being part of the invention, the present disclosure further relates to a method of delivering at least one substance to a vertebral body. Said method comprises the following steps:
- placing guiding means 22, such as a guiding wire, percutaneously through the skin to the final position of the bone screw 1 in a vertebra 3;
- guiding the system 2 comprising the bone screw 1 and at least three perforated catheters 21 along the guiding means 22 to the vertebra 3 and screwing the bone screw 1;
- removing the guiding means 22;
- selecting at least one catheter 21 which is in a blind channel 14 of interest;
- delivering at least one substance within the selected catheter 21 such that the at least one substance is discharged within the vertebral body in a chosen orientation;
- optionally, implanting a fluid pump fluidly connected to the selected catheter 21 subcutaneously.

In one example, the method comprises guiding extracting means along the flexible directing means 15 to the screw head 12 and extracting the bone screw 1 and the at least three catheters 21.

Not being part of the invention, the present disclosure further relates to a method of treating a disease, comprising delivering a therapeutically effective amount of at least one substance to a vertebral body. Said method comprises the following steps:
- placing guiding means 22, such as a guiding wire, percutaneously through the skin to the final position of the bone screw 1 in a vertebra 3;
- guiding the system 2 comprising the bone screw 1 and at least three perforated catheters 21 along the guiding means 22 to the vertebra 3 and screwing the bone screw 1;
- removing the guiding means 22;
- selecting at least one catheter 21 which is in a blind channel 14 of interest;
- delivering the therapeutically effective amount of at least one substance within the selected catheter 21 such that the at least one substance is discharged within the vertebral body in a chosen orientation;
- optionally, implanting a fluid pump fluidly connected to the selected catheter 21 subcutaneously.

In one example, the method comprises guiding extracting means along the flexible directing means 15 to the screw head 12 and extracting the bone screw 1 and the at least three catheters 21.

In one embodiment, the disease is selected from vertebral body metastasis, vertebral tumor, acute lumbar pain, intervertebral disc spondylodiscitis or degenerative disc disease.

Not being part of the invention, the present disclosure further relates to a method of delivering at least one substance to an intervertebral disc. Said method comprises the following steps:
- placing guiding means 22, such as a guiding wire, percutaneously through the skin to the final position of the bone screw 1 in a vertebra 3;
- guiding the system 2 comprising the bone screw 1 and at least three perforated catheters 21 along the guiding means 22 to the vertebra 3 and screwing the bone screw 1;
- removing the guiding means 22;
- selecting the catheter 21 which is in the blind channel 14 directed towards the superior or the inferior endplate;
- delivering at least one substance within the selected catheter 21 such that the at least one substance diffused towards respectively the superior or the inferior intervertebral disc;
- optionally, implanting a fluid pump fluidly connected to the selected catheter 21 subcutaneously.

In one example, the method comprises guiding extracting means along the flexible directing means 15 to the screw head 12 and extracting the bone screw 1 and the at least three catheters 21.

In one example, the method further comprises, prior to implantation of the at least one system 2, the step of accessing via transpedicular approach at least one endplate puncturing means 5 into a vertebral body; and puncturing respectively the superior or the inferior endplate.

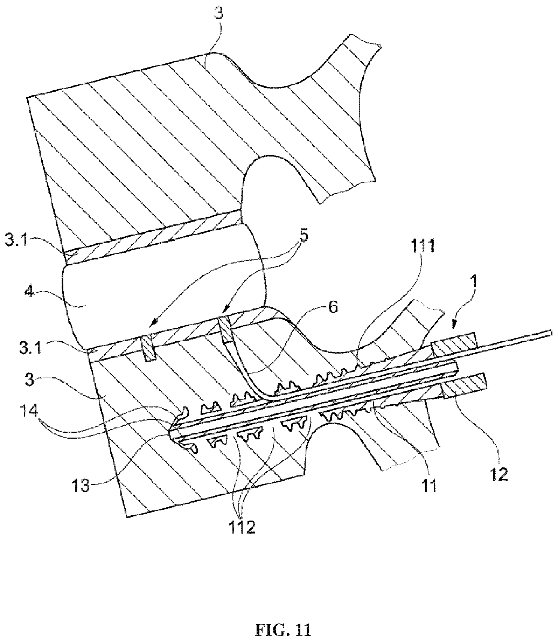

In one alternative example, as depicted in Fig. 11, the method further comprises, prior to the delivery of at least one substance within the selected catheter 21, the steps of:
- accessing via the selected blind channel 14 a steerable inserter 6 carrying an endplate puncturing means 5 at its distal end;
- steering the steerable inserter 6 in such a way that the inserter 6 exits out of the bone screw 1 through one fenestration 112;
- puncturing respectively the superior or the inferior endplate 3.1; and
- removing of the steerable inserter 6.

The steerable inserter 6 is advantageously a rod in shape-memory alloy, such as a Nitinol rod.

In one embodiment, from 1 to 5 endplate puncturing means 5 are engaged to the superior or inferior endplate through different fenestrations 112. Consequently, from 1 to 5 steerable inserters 6 are one after the other inserted in the selected blind channel 14 and punctured in the endplate.

According to one embodiment, the endplate puncturing means 5 is secured to the distal end of the steerable inserter 6 by welding, screwing or any other means known to one skilled in the art. According to one particular embodiment, in order to facilitate unfastening between the steerable inserter 6 and the endplate puncturing means 5 after insertion within the vertebral endplate, a torque is applied to the inserter 6.

As depicted in Fig. 12, each endplate puncturing means 5 creates a fluidic pathway from the intravertebral body directly towards the superior annulus fibrosus by means of the aperture 5.1; thereby reestablishing exchange of substances or nutrients. The engaging means 5.2 secures the endplate puncturing means 5 to the endplate. Once the endplate puncturing means 5 has been positioned as well as the bone screw 1, the catheter 21 which is in the blind channel 14 directed towards the endplate puncturing means 5 is selected and at least one substance is delivered through said selected catheter 21.

According to the Applicant, the combination of at least one endplate puncturing means 5 and the bone screw 1 of the invention with oriented delivery of substance is particularly advantageous in the treatment of intervertebral disc 4.

Not being part of the invention, the present disclosure further relates to a method of treating a disease, comprising delivering a therapeutically effective amount of at least one substance to an intervertebral disc. Said method comprises the following steps:
- placing guiding means 22, such as a guiding wire, percutaneously through the skin to the final position of the bone screw 1 in a vertebra 3;
- guiding the system 2 comprising the bone screw 1 and at least three perforated catheters 21 along the guiding means 22 to the vertebra 3 and screwing the bone screw 1;
- removing the guiding means 22;
- selecting the catheter 21 which is in the blind channel 14 directed towards the superior or the inferior endplate;
- delivering a therapeutically effective amount of at least one substance within the selected catheter 21 such that the therapeutically effective amount of the at least one substance diffused towards respectively the superior or the inferior intervertebral disc;
- optionally, implanting a fluid pump fluidly connected to the selected catheter 21 subcutaneously.

In one example, the method comprises guiding extracting means along the flexible directing means 15 to the screw head 12 and extracting the bone screw 1 and the at least three catheters 21.

In one example, the method further comprises, prior to implantation of the at least one system 2, the step of accessing via transpedicular approach at least endplate puncturing means 5 into a vertebral body; and puncturing respectively the superior or the inferior endplate.

In one alternative example, as depicted in Fig. 11, the method further comprises, prior to the delivery of at least one substance within the selected catheter 21, the steps of:
- accessing via the selected blind channel 14 a steerable inserter 6 carrying an endplate puncturing means 5 at its distal end;
- steering the steerable inserter 6 in such a way that the inserter 6 exits out of the bone screw 1 through one fenestration 112;
- puncturing respectively the superior or the inferior endplate; and
- removing of the steerable inserter 6.

In one example, the disease is selected from vertebral body metastasis, vertebral tumor, acute lumbar pain, intervertebral disc spondylodiscitis or degenerative disc disease.

In one example, the bone screw 1 is implanted via transpedicular approach.

In one embodiment, the system comprises a perforated catheter 21 in each blind channel 14.

In one embodiment, the system comprises a stopper 23.

According to one embodiment, the substances delivered are chemotherapeutics to treat vertebral body metastasis or vertebral tumor. According to another embodiment, the substances delivered are anesthetics to treat acute lumbar pain. According to one embodiment, the substances delivered are antibiotics to treat intervertebral disc spondylodiscitis. According to one embodiment, the substances delivered are nutrients to ensure intervertebral regeneration. According to one embodiment, the substances delivered are growth factors, non-steroid drugs, steroids, anti TNF-α to treat degenerative disc disease. According to one embodiment, the substances delivered are anesthetics to treat acute lumbar pain.

The present invention further relates to a method of treating a vertebra 3; said method comprising the steps of:
- placing guiding means 22, such as a guiding wire, percutaneously through the skin to the final position of the bone screw 1 in a vertebra 3;
- guiding the system 2 comprising the bone screw 1 and at least one radioactive needle along the guiding means 22 to the vertebra 3 and screwing the bone screw 1;
- removing the guiding means 22;
- irradiating the vertebra 3; and
- optionally, guiding extracting means along the flexible directing means 15 to the screw head 12 and extracting the bone screw 1 and the at least one needle.

In one example, said method is useful for treating vertebral body metastasis or vertebral tumor.

In one embodiment, the system comprises a radioactive needle in each blind channel 14.

In one embodiment, the system comprises a stopper 23.

While various embodiments have been described and illustrated, the detailed description is not to be construed as being limited hereto.

### BRIEF DESCRIPTION OF THE DRAWINGS

**Figure 1** shows the system comprising two catheters according to one embodiment of the invention.
**Figure 2** is a sectional view of a bone screw according to one embodiment of the present invention in a vertebra.
**Figure 3A** is sectional view of the screw body in a transverse plane wherein the bone screw comprises three blind channels.
**Figure 3B** is sectional view of the screw body in a transverse plane wherein the bone screw comprises four blind channels.
**Figure 4** is a partial side view of the bone screw according to one embodiment of the present invention wherein the flexible directing means are a braided wire.
**Figure 5** is a partial side view of the bone screw according to the present invention wherein the flexible directing means are a braided sleeve.
**Figure 6** is a partial side view of the bone screw according to one embodiment of the present invention wherein the flexible directing means are a coiled sleeve.
**Figure 7A** is a partial front view of the bone screw according to one embodiment of the present invention.
**Figure 7B** is a partial front view of the system comprising four catheters and guiding means according to one embodiment of the present invention.
**Figure 8** illustrates the stopper according to one embodiment of the present invention.
**Figure 9** is a sectional view of a system comprising at least two catheters according to one embodiment of the present invention in a vertebra.
**Figure 10** shows the bone screw according to one embodiment of the invention.
**Figure 11** illustrates the kit of parts in use during positioning of an endplate puncturing means according to one embodiment of the invention.
**Figure 12** illustrates the kit of parts in use after insertion of the endplate puncturing means according to one embodiment of the invention.
**Figures 13A** illustrates the endplate puncturing means according to one embodiment of the invention.
**Figures 13B** illustrates a sectional view of the endplate puncturing means according to a first embodiment of the invention.
**Figures 13C** illustrates a sectional view of the endplate puncturing means according to a second embodiment of the invention.

### REFERENCES

1 - Bone screw
11 - Screw body
111 - Thread
112 - Fenestration
12 - Screw head
13 - Through bore
14 - Blind channel
15 - Directing means
151 - Braided sleeve
152 - Coiled sleeve
153 - Wire
2 - System
21 - Catheter
22 - Guiding means
23 - Stopper
231 - O-ring
232 - Guiding tube
2321 - O-ring
3 - Vertebra
3.1 - Endplate
4 - Disc
5 - Endplate puncturing means
5.1 - Aperture
5.2 - Engaging means
6 - Steerable inserter

## Claims

1. A bone screw (1) comprising a screw body (11) comprising a proximal end, a distal end, a longitudinal axis and a thread (111) along all or a portion of said screw body (11); a screw head (12) attached to the proximal end of the screw body (11), **characterized in that** it comprises :
- a through bore (13) extending coaxially through said screw head (12) and through said screw body (11) from the proximal end up to the distal end;
- at least three blind channels (14) angularly-disposed around the through bore (13); said blind channels (14) extending longitudinally through said screw head (12) and through a portion of said screw body (11); and each of said blind channels (14) being configured to receive a catheter (21) or a needle; and
- a plurality of radially-disposed fenestrations (112); wherein each of said blind channels (14) is connected to an exterior of said screw body (11) through at least one fenestration (112).

2. The bone screw (1) according to claim 1, wherein the at least three blind channels (14) are regularly angularly disposed.

3. The bone screw (1) according to claim 1 comprising four blind channels (14) disposed at every 90°.

4. The bone screw (1) according to claim 1, wherein at least one of the blind channels (14) has a different length than the other channels.

5. The bone screw (1) according to claim 1, wherein the fenestrations (112) are elongated holes.

6. The bone screw (1) according to claim 1, wherein the fenestrations (112) are positioned between adjacent crests of the thread (111).

7. The bone screw (1) according to claim 1, wherein a fenestration (112) is located at the distal end of each blind channel (14).

8. The bone screw (1) according to claim 1, wherein the plurality of radially-disposed fenestrations (112) are longitudinally spaced.

9. The bone screw (1) according to claim 1, further comprising a radiopaque marker enabling angular positioning of the bone screw (1) within the bone.

10. The bone screw (1) according to claim 1, further comprising flexible directing means (15) secured to the screw head (12) such that extracting means may be guided to the screw head (12) along said flexible directing means (15).

11. The bone screw (1) according to claim 10, wherein the flexible directing means (15) is a wire (153).

12. The bone screw (1) according to claim 10, wherein the flexible directing means (15) is hollow and configured to receive at least one catheter (21) or at least one needle.

13. The bone screw (1) according to claim 10, wherein the flexible directing means (15) comprises a braided sleeve (151) configured to receive at least one catheter (21) or at least one needle.

14. The bone screw (1) according to claim 10, wherein the flexible directing means (15) comprises a coiled sleeve (152) configured to receive at least one catheter (21) or at least one needle.

15. The bone screw (1) according to claim 1, wherein the lead of the thread (111) varies along the screw body (11).

16. The bone screw (1) according to claim 1, wherein the lead of the thread (111) varies along the screw body (11) and wherein the lead of the thread (111) is larger in the distal portion of the screw body (11) than in the proximal portion of the screw body (11).

17. A system (2) comprising:
a bone screw (1) comprising
- a screw body (11) comprising a proximal end, a distal end, a longitudinal axis and a thread (111) along all or a portion of said screw body (11);
- a screw head (12) attached to the proximal end of the screw body (11), **characterized in that** it comprises :
- a through bore (13) extending coaxially through said screw head (12) and through said screw body (11) from the proximal end up to the distal end;
- at least three blind channels (14) angularly disposed around the through bore (13); said blind channels (14) extending longitudinally through said screw head (12) and through a portion of said screw body (11); and each of said blind channels (14) being configured to receive a catheter (21) or a needle; and
- a plurality of radially-disposed fenestrations (112); wherein each of said blind channels (14) is connected to an exterior of said screw body (11) through at least one fenestration (112); and at least one perforated catheter or at least one radioactive needle.

18. The system according to claim 17, further comprising flexible directing means (15) secured to the screw head (12) such that extracting means may be guided to the screw head (12) along said flexible directing means (15).

19. The system according to claim 17, further comprising a stopper (23) comprising an opening for each catheter or for each needle; wherein said stopper (23) is configured to be inserted in the screw head in a tight manner.

20. The system according to claim 17 comprising at least one perforated catheter (21) and further comprising a subcutaneous fluid pump fluidly connected to the at least one perforated catheter (21).

21. A kit of parts comprising:
a bone screw (1) comprising a screw body (11) comprising a proximal end, a distal end, a longitudinal axis and a thread (111) along all or a portion of said screw body (11); a screw head (12) attached to the proximal end of the screw body (11), **characterized in that** it comprises :
- a through bore (13) extending coaxially through said screw head (12) and through said screw body (11) from the proximal end up to the distal end;
- at least three blind channels (14) angularly disposed around the through bore (13); said blind channels (14) extending longitudinally through said screw head (12) and through a portion of said screw body (11); and each of said blind channels (14) being configured to receive a catheter (21) or a needle; and
- a plurality of radially-disposed fenestrations (112); wherein each of said blind channels (14) is connected to an exterior of said screw body (11) through at least one fenestration (112); and
at least one endplate puncturing means (5).

22. The kit of parts according to claim 21, wherein the endplate puncturing means (5) comprises at least one aperture (5.1) extending therethrough; and engaging means (5.2) configured to mechanically engaged the endplate of a vertebra.

23. The kit of parts according to claim 21, wherein the aperture (5.1) extends from a distal end to a proximal end along a longitudinal axis.

24. The kit of parts according to claim 21, wherein the aperture (5.1) extends from a side opening adjacent to a distal end to a proximal end.

25. The kit of parts according to claim 21, wherein the distal end is blunted so as to prevent disc perforation.

26. The kit of parts according to claim 21, wherein the length of the endplate puncturing means (5) from a distal end to a proximal end along a longitudinal axis is lower than 5 mm.

27. The kit of parts according to claim 21, wherein the endplate puncturing means (5) comprises a radiopaque marker at its distal end.

## Patentansprüche

1. Eine Knochenschraube (1), umfassend einen Schraubenkörper (11), umfassend ein proximales Ende, ein distales Ende, eine Längsachse und ein Gewinde (111) entlang des gesamten oder eines Abschnitts des Schraubenkörpers (11); einen Schraubenkopf (12), der an dem proximalen Ende des Schraubenkörpers (11) befestigt ist, **dadurch gekennzeichnet, dass** er aufweist:
- eine Durchgangsbohrung (13), die sich koaxial durch den Schraubenkopf (12) und durch den Schraubenkörper (11) vom proximalen Ende bis zum distalen Ende erstreckt,
- mindestens drei Blindkanäle (14), die winkelig um die Durchgangsbohrung (13) angeordnet sind; wobei sich die Blindkanäle (14) in Längsrichtung durch den Schraubenkopf (12) und durch einen Abschnitt des Schraubenkörpers (11) erstrecken und wobei jeder der Blindkanäle (14) konfiguriert ist, um einen Katheter (21) oder eine Nadel aufzunehmen, und
- mehrere radial angeordnete Fensterungen (112), wobei jeder der Blindkanäle (14) durch mindestens eine Fensterung (112) mit einer Außenseite des Schraubenkörpers (11) verbunden ist.

2. Die Knochenschraube (1) nach Anspruch 1, wobei die mindestens drei Blindkanäle (14) regelmäßig winkelig angeordnet sind.

3. Die Knochenschraube (1) nach Anspruch 1, umfassend vier Blindkanäle (14), die um jeweils 90° angeordnet sind.

4. Die Knochenschraube (1) nach Anspruch 1, wobei mindestens einer der Blindkanäle (14) eine andere Länge als die anderen Kanäle aufweist.

5. Die Knochenschraube (1) nach Anspruch 1, wobei die Fensterungen (112) Langlöcher sind.

6. Die Knochenschraube (1) nach Anspruch 1, wobei die Fensterungen (112) zwischen benachbarten Spitzen des Gewindes (111) positioniert sind.

7. Die Knochenschraube (1) nach Anspruch 1, wobei sich am distalen Ende jedes Blindkanals (14) eine Fensterung (112) befindet.

8. Die Knochenschraube (1) nach Anspruch 1, wobei die mehreren radial angeordneten Fensterungen (112) in Längsrichtung beabstandet sind.

9. Die Knochenschraube (1) nach Anspruch 1, ferner umfassend eine röntgendichte Markierung, die eine Winkelpositionierung der Knochenschraube (1) im Knochen ermöglicht.

10. Die Knochenschraube (1) nach Anspruch 1, ferner umfassend ein flexibles Ausrichtungsmittel (15), das an dem Schraubenkopf (12) derart befestigt sind, dass ein Extraktionsmittel entlang des flexiblen Ausrichtungsmittels (15) zum Schraubenkopf (12) geführt werden kann.

11. Die Knochenschraube (1) nach Anspruch 10, wobei das flexible Ausrichtungsmittel (15) ein Draht (153) ist.

12. Die Knochenschraube (1) nach Anspruch 10, wobei das flexible Ausrichtungsmittel (15) hohl und konfiguriert ist, um mindestens einen Katheter (21) oder mindestens eine Nadel aufzunehmen.

13. Die Knochenschraube (1) nach Anspruch 10, wobei das flexible Ausrichtungsmittel (15) eine geflochtene Hülse (151) aufweist, die konfiguriert ist, um mindestens einen Katheter (21) oder mindestens eine Nadel aufzunehmen.

14. Die Knochenschraube (1) nach Anspruch 10, wobei das flexible Ausrichtungsmittel (15) eine gewickelte Hülse (152) aufweist, die konfiguriert ist, um mindestens einen Katheter (21) oder mindestens eine Nadel aufzunehmen.

15. Die Knochenschraube (1) nach Anspruch 1, wobei die Steigung des Gewindes (111) entlang des Schraubenkörpers (11) variiert.

16. Die Knochenschraube (1) nach Anspruch 1, wobei die Steigung des Gewindes (111) entlang des Schraubenkörpers (11) variiert und wobei die Steigung des Gewindes (111) im distalen Abschnitt des Schraubenkörpers (11) größer ist als im proximalen Abschnitt des Schraubenkörpers (11).

17. Ein System (2), umfassend:
eine Knochenschraube (1), umfassend
- einen Schraubenkörper (11), umfassend ein proximales Ende, ein distales Ende, eine Längsachse und ein Gewinde (111) entlang des gesamten oder eines Abschnitts des Schraubenkörpers (11),
- einen Schraubenkopf (12), der an dem proximalen Ende des Schraubenkörpers (11) befestigt ist, **dadurch gekennzeichnet, dass** er aufweist:
- eine Durchgangsbohrung (13), die sich koaxial durch den Schraubenkopf (12) und durch den Schraubenkörper (11) vom proximalen Ende bis zum distalen Ende erstreckt;
- mindestens drei Blindkanäle (14), die winkelig um die Durchgangsbohrung (13) angeordnet sind, wobei sich die Blindkanäle (14) in Längsrichtung durch den Schraubenkopf (12) und durch einen Abschnitt des Schraubenkörpers (11) erstrecken und wobei jeder der Blindkanäle (14) konfiguriert ist, um einen Katheter (21) oder eine Nadel aufzunehmen, und
- mehrere radial angeordnete Fensterungen (112), wobei jeder der Blindkanäle (14) durch mindestens eine Fensterung (112) mit einer Außenseite des Schraubenkörpers (11) verbunden ist, und mindestens einen perforierten Katheter oder mindestens eine radioaktive Nadel.

18. Das System nach Anspruch 17, ferner umfassend ein flexibles Ausrichtungsmittel (15), das an dem Schraubenkopf (12) derart befestigt sind, dass ein Extraktionsmittel entlang des flexiblen Ausrichtungsmittels (15) zum Schraubenkopf (12) geführt werden kann.

19. Das System nach Anspruch 17, ferner umfassend einen Stopfen (23), umfassend eine Öffnung für jeden Katheter oder für jede Nadel,
wobei der Stopfen (23) konfiguriert ist, um dichtend in den Schraubenkopf eingesetzt zu werden.

20. Das System nach Anspruch 17, umfassend mindestens einen perforierten Katheter (21) und ferner umfassend eine subkutane Fluidpumpe, die mit dem mindestens einen perforierten Katheter (21) fluidisch verbunden ist.

21. Ein Teilesatz, umfassend:
eine Knochenschraube (1), umfassend einen Schraubenkörper (11), umfassend ein proximales Ende, ein distales Ende, eine Längsachse und ein Gewinde (111) entlang des gesamten oder eines Abschnitts des Schraubenkörpers (11); einen Schraubenkopf (12), der an dem proximalen Ende des Schraubenkörpers (11) befestigt ist, **dadurch gekennzeichnet, dass** er aufweist:
- eine Durchgangsbohrung (13), die sich koaxial durch den Schraubenkopf (12) und durch den Schraubenkörper (11) vom proximalen Ende bis zum distalen Ende erstreckt,
- mindestens drei Blindkanäle (14), die winkelig um die Durchgangsbohrung (13) angeordnet sind, wobei sich die Blindkanäle (14) in Längsrichtung durch den Schraubenkopf (12) und durch einen Abschnitt des Schraubenkörpers (1) erstrecken und
wobei jeder der Blindkanäle (14) konfiguriert ist, um einen Katheter (21) oder eine Nadel aufzunehmen, und
- mehrere radial angeordnete Fensterungen (112), wobei jeder der Blindkanäle (14) durch mindestens eine Fensterung (112) mit einer Außenseite des Schraubenkörpers (1) verbunden ist, und
mindestens ein Endplatten-Durchstechmittel (5).

22. Der Teilesatz nach Anspruch 21, wobei das Endplatten-Durchstechmittel (5) mindestens eine Öffnung (5.1), die sich dadurch erstreckt, und Eingriffsmittel (5.2) aufweist, die konfiguriert sind, um die Endplatte eines Wirbels mechanisch in Eingriff zu bringen.

23. Der Teilesatz nach Anspruch 21, wobei sich die Öffnung (5.1) von einem distalen Ende zu einem proximalen Ende entlang einer Längsachse erstreckt.

24. Der Teilesatz nach Anspruch 21, wobei sich die Öffnung (5.1) von einer seitlichen Öffnung neben einem distalen Ende zu einem proximalen Ende erstreckt.

25. Der Teilesatz nach Anspruch 21, wobei das distale Ende derart abgestumpft ist, um eine Bandscheibenperforation zu verhindern.

26. Der Teilesatz nach Anspruch 21, wobei die Länge des Endplatten-Durchstechmittels (5) von einem distalen Ende zu einem proximalen Ende entlang einer Längsachse kleiner als 5 mm ist.

27. Der Teilesatz nach Anspruch 21, wobei das Endplatten-Durchstechmittel (5) an seinem distalen Ende eine röntgendichte Markierung aufweist.

## Revendications

1. Une vis à os (1) comprenant un corps de vis (11) comprenant une extrémité proximale, une extrémité distale, un axe longitudinal et un filetage (111) le long de tout ou partie dudit corps de vis (11) ; une tête de vis (12) fixée à l'extrémité proximale du corps de vis (11), **caractérisée en ce qu'**elle comprend :
- un alésage traversant (13) s'étendant coaxialement à travers ladite tête de vis (12) et à travers ledit corps de vis (11) depuis l'extrémité proximale jusqu'à l'extrémité distale ;
- au moins trois canaux borgnes (14) disposés angulairement autour de l'alésage traversant (13) ; lesdits canaux borgnes (14) s'étendant longitudinalement à travers ladite tête de vis (12) et à travers une partie dudit corps de vis (11) ; et chacun desdits canaux borgnes (14) étant configuré pour recevoir un cathéter (21) ou une aiguille ; et
- une pluralité de fenêtres (112) disposées radialement ; chacun desdits canaux borgnes (14) étant relié à l'extérieur dudit corps de vis (11) par au moins une fenêtre (112).

2. La vis à os (1) selon la revendication 1, dans laquelle lesdits au moins trois canaux borgnes (14) sont régulièrement disposés angulairement.

3. La vis à os (1) selon la revendication 1, comprenant quatre canaux borgnes (14) disposés tous les 90°.

4. La vis à os (1) selon la revendication 1, dans laquelle au moins un des canaux borgnes (14) a une longueur différente des autres canaux.

5. La vis à os (1) selon la revendication 1, dans laquelle les fenêtres (112) sont des trous allongés.

6. La vis à os (1) selon la revendication 1, dans laquelle les fenêtres (112) sont positionnées entre des crêtes adjacentes du filetage (111).

7. La vis à os (1) selon la revendication 1, dans laquelle une fenêtre (112) est située à l'extrémité distale de chaque canal borgne (14).

8. La vis à os (1) selon la revendication 1, dans laquelle les fenêtres de la pluralité de fenêtres disposées radialement (112) sont espacées longitudinalement.

9. La vis à os (1) selon la revendication 1, comprenant en outre un marqueur radio-opaque permettant le positionnement angulaire de la vis à os (1) à l'intérieur de l'os.

10. La vis à os (1) selon la revendication 1, comprenant en outre un moyen directionnel flexible (15) fixé à la tête de vis (12), tel que des moyens d'extraction peuvent être guidés vers la tête de vis (12) le long dudit moyen directionnel flexible (15).).

11. La vis à os (1) selon la revendication 10, dans laquelle le moyen directionnel flexible (15) est un fil (153).

12. La vis à os (1) selon la revendication 10, dans laquelle le moyen directionnel flexible (15) est creux et configuré pour recevoir au moins un cathéter (21) ou au moins une aiguille.

13. La vis à os (1) selon la revendication 10, dans laquelle le moyen directionnel flexible (15) comprend un manchon tressé (151) configuré pour recevoir au moins un cathéter (21) ou au moins une aiguille.

14. La vis à os (1) selon la revendication 10, dans laquelle le moyen directionnel flexible (15) comprend un manchon enroulé (152) configuré pour recevoir au moins un cathéter (21) ou au moins une aiguille.

15. La vis à os (1) selon la revendication 1, dans laquelle le pas du filetage (111) varie le long du corps de vis (11).

16. La vis à os (1) selon la revendication 1, dans laquelle le pas du filetage (111) varie le long du corps de vis (11) et dans laquelle le pas du filetage (111) est plus grand dans la partie distale du corps de vis (11) que dans la partie proximale du corps de vis (11).

17. Un système (2) comprenant :
une vis à os (1) comprenant :
- un corps de vis (11) comprenant une extrémité proximale, une extrémité distale, un axe longitudinal et un filetage (111) le long de tout ou partie dudit corps de vis (11) ;
- une tête de vis (12) fixée à l'extrémité proximale du corps de vis (11), **caractérisée en ce qu'**elle comprend :
- un alésage traversant (13) s'étendant coaxialement à travers ladite tête de vis (12) et à travers ledit corps de vis (11) depuis l'extrémité proximale jusqu'à l'extrémité distale ;
- au moins trois canaux borgnes (14) disposés angulairement autour de l'alésage traversant (13) ; lesdits canaux borgnes (14) s'étendant longitudinalement à travers ladite tête de vis (12) et à travers une partie dudit corps de vis (11) ; et chacun desdits canaux borgnes (14) étant configuré pour recevoir un cathéter (21) ou une aiguille ; et
- une pluralité de fenêtres (112) disposées radialement; chacun desdits canaux borgnes (14) est relié à l'extérieur dudit corps de vis (11) par au moins une fenêtre (112) ; et au moins un cathéter perforé ou au moins une aiguille radioactive.

18. Le système selon la revendication 17, comprenant en outre un moyen directionnel flexible (15) fixé à la tête de vis (12), tel que des moyens d'extraction peuvent être guidés vers la tête de vis (12) le long dudit moyen directionnel flexible (15).

19. Le système selon la revendication 17, comprenant en outre un organe d'arrêt (23) comprenant une ouverture pour chaque cathéter ou pour chaque aiguille ; ledit organe d'arrêt (23) est configuré pour être inséré dans la tête de vis d'une manière serrée.

20. Le système selon la revendication 17, comprenant au moins un cathéter perforé (21) et comprenant en outre une pompe à fluide sous-cutanée reliée fluidiquement audit au moins un cathéter perforé (21).

21. Un kit de pièces comprenant :
une vis à os (1) comprenant un corps de vis (11) comprenant une extrémité proximale, une extrémité distale, un axe longitudinal et un filetage (111) le long de tout ou partie dudit corps de vis (11) ; une tête de vis (12) fixée à l'extrémité proximale du corps de vis (11), **caractérisée en ce qu'**elle comprend :
- un alésage traversant (13) s'étendant coaxialement à travers ladite tête de vis (12) et à travers ledit corps de vis (11) depuis l'extrémité proximale jusqu'à l'extrémité distale ;
- au moins trois canaux borgnes (14) disposés angulairement autour de l'alésage traversant (13) ; lesdits canaux borgnes (14) s'étendant longitudinalement à travers ladite tête de vis (12) et à travers une partie dudit corps de vis (11) ; et chacun desdits canaux borgnes (14) étant configuré pour recevoir un cathéter (21) ou une aiguille ; et
- une pluralité de fenêtres (112) disposées radialement ; chacun desdits canaux borgnes (14) est relié à l'extérieur dudit corps de vis (11) par au moins une fenêtre (112) ; et
au moins un moyen (5) de perforation de plateau d'extrémité.

22. Le kit de pièces selon la revendication 21, dans lequel le moyen (5) de perforation de plateau d'extrémité comprend au moins une ouverture (5.1) s'étendant à travers lui ; et des moyens d'engagement (5.2) configurés pour venir mécaniquement en engagement avec le plateau d'extrémité d'une vertèbre.

23. Le kit de pièces selon la revendication 21, dans lequel l'ouverture (5.1) s'étend depuis une extrémité distale jusqu'à une extrémité proximale le long d'un axe longitudinal.

24. Le kit de pièces selon la revendication 21, dans lequel l'ouverture (5.1) s'étend depuis une ouverture latérale adjacente à une extrémité distale jusqu'à une extrémité proximale.

25. Le kit de pièces selon la revendication 21, dans lequel l'extrémité distale est émoussée de manière à éviter la perforation du disque.

26. Le kit de pièces selon la revendication 21, dans lequel la longueur des moyens (5) de perforation de plateau d'extrémité depuis une extrémité distale jusqu'à une extrémité proximale selon un axe longitudinal est inférieure à 5 mm.

27. Le kit de pièces selon la revendication 21, dans lequel le moyen (5) de perforation de plateau d'extrémité comprend un marqueur radio-opaque à son extrémité distale.
